# EUROPÄISCHE PATENTSCHRIFT

(11) **EP 0 450 587 B1**
(45) Veröffentlichungstag und Bekanntmachung des Hinweises auf die Patenterteilung: **09.07.1997**
(21) Anmeldenummer: 91105235.5
(22) Anmeldetag: 03.04.1991
(51) Int. Cl.: C07D 209/48, C11D 3/39

(54) **Stabile Peroxicarbonsäuregranulate**
Stable granules of peroxycarboxylic acid
Granulés stables de peracides carboxyliques

(30) Priorität: 06.04.1990 DE 4011185
(43) Veröffentlichungstag der Anmeldung: 09.10.1991
(73) Patentinhaber: HOECHST AKTIENGESELLSCHAFT, 65926 Frankfurt am Main (DE)
(72) Erfinder: Gethöffer, Hanspeter, Dr., I-10151 Turin (IT); Reinhardt, Gerd, Dr., W-6233 Kelkheim/Taunus (DE); Nöltner, Gerhard, W-6230 Frankfurt am Main (DE); Funk, Rüdiger, Dr., W-6200 Wiesbaden (DE)

(56) Entgegenhaltungen:
- EP-A- 0 256 443
- EP-A- 0 272 402
- EP-A- 0 349 940
- EP-A- 0 360 323

## Beschreibung

Die vorliegende Erfindung betrifft lagerstabile, hochprozentige Bleichwirkstoffe in granulierter Form, die als Bleichkomponenten feste Imidoperoxicarbonsäuren enthalten. Die erfindungsgemäßen Granulate können als Bleichadditive oder Oxidationsmittel in Wasch-, Reinigungs- und Desinfektionsmitteln eingesetzt werden.

Anorganische Persalze wie Natriumperborat oder Percarbonate sind seit langem als Bleichmittelzusätze in Waschmitteln bekannt. Ihre optimale Bleichkraft entfalten sie jedoch erst bei Temperaturen oberhalb von 60°C. Zu ihrer Aktivierung sind eine Reihe organischer Verbindungen beschrieben, die während des Waschprozeßes mit Wasserstoffperoxid eine Peroxicarbonsäure freisetzen. Diese wirkt bereits bei Temperaturen unterhalb von 60°C bleichend. Bekanntestes Beispiel dafür ist Tetraacetylethylendiamin (TAED).

Daneben werden jedoch in neuerer Zeit auch eine Reihe von Peroxicarbonsäuren für den direkten Einsatz in Waschmitteln beschrieben.

Problematisch ist jedoch sowohl bei den Aktivatoren als auch bei den vorgefertigten Peroxicarbonsäuren ihre geringe Lagerstabilität in alkalischen Waschmittelformulierungen. Eine ausreichende Lagerstabilität kann bei diesen Substanzen nur durch ein geeignetes Granulier- bzw. Coatingverfahren erreicht werden.

Als Granulierhilfsmittel für den am häufigsten benutzten Persalzaktivator Tetraacetylethylendiamin sind z.B. Carboximethylcellulose oder Ethoxilate längerkettiger Alkohole bekannt.

Reaktivere Persalzaktivatoren wie Phthalsäureanhydrid hingegen benötigen einen effektiveren Schutz. So wird zur Herstellung lagerstabiler Granulate ein vorgefertigtes Aktivatorgranulat, bestehend aus Phthalsäureanhydrid und einem Trägermaterial, mit einem Umhüllungsmaterial aus polymeren organischen Verbindungen wie Polyacrylamid, Copolymeren von Acrylsäure, Methacrylsäure oder Maleinsäureanhydrid bzw. Stärke oder Celluloseethern umhüllt (US 4.009.113).

Die Stabilisierung anderer empfindlicher Waschmittelkomponenten (Enzyme oder Percarbonate) durch Umhüllen mit polymeren Materialien gehört inzwischen zum Stand der Technik.

Ein besonderes Problem ist jedoch auch heutzutage noch die Stabilisierung reaktiver Peroxicarbonsäuren. In Gegenwart basischer Waschmittelbestandteile, Parfüme und Enzyme kommt es leicht zu Redoxreaktionen unter Verlust von Aktivsauerstoff. Daneben kann es sehr leicht zu Oxidationsreaktionen kommen, bei denen wertvolle Waschmittelkomponenten wie Parfüme oder Enzyme oxidativ zerstört werden.

Zur Lösung des Problems sind eine Reihe von Vorschlägen gemacht worden.

So wird in der EP 200.163 ein einheitlich zusammengesetztes Granulat, bestehend aus 3-50 % einer aliphatischen Peroxicarbonsäure, 40-95 % eines hydratisierbaren anorganischen Salzes und 0,2 - 10 % einer organischen Polymerverbindung wie Polyacrylsäure, beschrieben.

Ein Granulat mit einer Partikelgröße von 0,5 bis 2 mm, bestehend aus 20 - 65 % einer Peroxicarbonsäure, 30 - 79,5 % eines anorganischen Salzes und 0,5 - 6,5 % einer polymeren Säure als Binder ist in EP 256.443 beschrieben. Das Produkt kann in einem zusätzlichen Reaktionsschritt mit einem Überzugsmaterial umhüllt und so vor Reaktionen mit oxidierbaren Waschmittelbestandteilen geschützt werden.

Ein analoges Granulat und seine Herstellung werden in EP 272.402 beschrieben. Dabei wird ein vorgefertigtes Peroxicarbonsäuregranulat unter Bewegung mit einer wäßrigen Lösung eines im alkalischen Milieu löslichen Homo- oder Copolymerisats einer ungesättigten, 3 - 6 Kohlenstoffatome enthaltenden organischen Carbonsäure besprüht und gleichzeitig oder anschließend getrocknet. Bevorzugte vorgefertigfe Granulate bestehen aus 3 - 50, insbesondere 7 - 20 % einer Peroxicarbonsäure, wobei wiederum α,ω-Dodecandipersäure bevorzugt wird.

Weiterhin sind Granulate aus festen, bevorzugt aliphatischen Peroxicarbonsäurepartikeln, die mit oberflächenaktiven Substanzen überzogen sind, beschrieben. (DOS 2737864). Zur Steuerung einer exothermen Zersetzungsreaktion kann das überzogene Peroxicarbonsäureteilchen noch mit anorganischen Sulfaten vereinigt werden. Außerdem kann zum weiteren Schutz des Granulates eine zusätzliche Umhüllung des Granulatkernes mit Säure-, Ester-, Ether-oder Kohlenwasserstoff-haltigen Substanzen durchgeführt werden. Diese Materialien tragen dazu bei, daß Feuchtigkeit davon abgehalten wird, die Peroxicarbonsäureteilchen zu erreichen.

In den Patentanmeldungen EP 200.163 und EP 272.402 wird ausdrücklich darauf hingewiesen, daß sich die Erfahrungen, die mit einem Peroxicarbonsäuretyp gewonnen wurden, nur selten auf einen anderen Typ übertragen lassen. Optimale Granulate sind demnach nur durch Maßnahmen zu erreichen, die auf den jeweiligen Typ von Peroxicarbonsäure zugeschnitten sind. So ist z.B. aus US 3.639.285 bekannt, daß Tenside die Zersetzung von Peroxicarbonsäuren begünstigen, während sie in DOS 27 37 864 problemlos als Granulierhilfsmittel eingesetzt werden.

In den meisten bisher beschriebenen Granulaten wird als organische Persäure α,ω-Dodecandipersäure (DPDDA) eingesetzt. Aufgrund ihrer thermischen Instabilität kann sie nur in phlegmatisierter Form mit einem Gehalt bis zu 30 % in ein lagerstabiles Granulat überführt werden.

Weitere Granulate von Imidoperoxicarbonsäuren sind in EP-A-0 349 940 und EP-A-0 360 323 beschrieben.

Lagerstabile Granulate reaktiverer Persäuren mit Aktivgehalten von über 60 % sind bisher kaum beschrieben worden und stellen hohe Ansprüche an die Granuliertechnik.

Mit den Imidoperoxicarbonsäuren (EP 325288 und 349940) wurde eine Gruppe von Peroxicarbonsäuren entwickelt, die eine deutlich höhere Oxidations- und Bleichkraft als α,ω-Dodecandipersäure aufweist. Wirtschaftlich und anwendungstechnisch von besonderem Interesse ist ε-Phthalimidoperoxicapronsäure (PAP).

Ziel der vorliegenden Erfindung war es, diese Verbindungsklasse in ein geeignetes, lagerstabiles Granulat mit Aktivgehalten von mindestens 60 % zu überführen.

Die Aufgabe wird dadurch gelöst, daß die Imidoperoxicarbonsäure mit einem Granulierhilfsmittel in einem Mischer agglomeriert und das Agglomerat anschließend mit einem filmbildenden Mittel überzogen wird. Auf den Einsatz von Mitteln zur thermischen Stabilisierung der Persäure kann in diesem Fall verzichtet werden.

Gegenstand der Erfindung sind daher lagerstabile Peroxicarbonsäuregranulate, bestehend im wesentlichen aus einer Imidoperoxicarbonsäure oder deren Salze der Formel 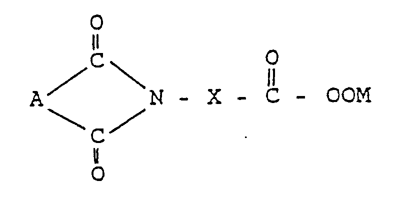 worin A eine Gruppe der Formeln 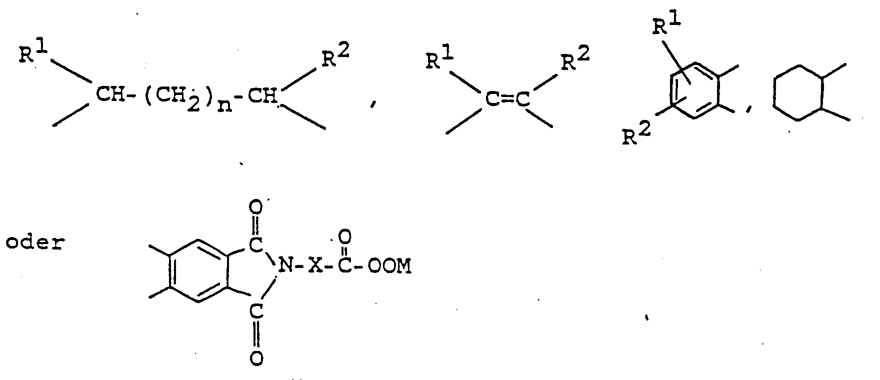
- n: die Zahlen 0, 1 oder 2,
- R¹: Wasserstoff, Chlor, Brom C₁-C₂₀-Alkyl, Alkenyl mit bis zu 20 C-Atomen, Aryl oder Alkylaryl.
- R²: Wasserstoff, Chlor, Brom oder eine Gruppe der Formel -SO₃M, -CO₂M oder -OSO₃M,
- M: Wasserstoff, ein Alkali- oder Ammonium-Ion oder das Äquivalent eines Erdalkali-Ions, und
- X: C₁-C₁₉-, bevorzugt C₃-C₁₁-Alkylen, oder Arylen, bevorzugt Phenylen bedeuten,
einem anorganischen Sulfat- und/oder Phosphat-Salz und/oder einem nicht oxidierbaren Tensid als Granulierhilfsmittel und einem Homo- oder Copolymer aus (Meth)acrylsäure oder deren Salzen oder einem Copolymer aus (Meth)acrylsäure oder deren Salzen und anderen hiermit polymerisierbaren organischen Carbonsäuren als filmbildende Hüllsubstanz, wobei der Gehalt an Imidoperoxicarbonsäure in dem Granulat mindestens 60 Gew. % beträgt.

Die drei wesentlichen Komponenten des erfindungsgemäßen Bleichmittels sind daher eine Peroxicarbonsäure aus der Gruppe der Imidoperoxicarbonsäuren, ein Granulierhilfsmittel und das Überzugsmittel. Diese werden, zusammen mit wahlweise zu verwendenden Komponenten, im Folgenden beschrieben.

### Die Peroxicarbonsäure

Als Peroxicarbonsäuren kommen die Imidoperoxicarbonsäuren der oben angegebenen Formel in Frage. Bevorzugt sind solche Verbindungen dieser Formel, worin
- A: eine Gruppe der Formel
-CH₂-(CH₂)ₙ-CH₂- oder -CH₂-CHR¹-, 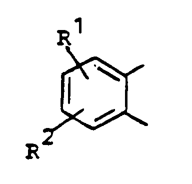
- n: die Zahlen 0 oder 1,
- R¹: Wasserstoff, C₁-C₂₀-Alkyl oder C₁-C₂₀-Alkenyl,
- R²: Wasserstoff oder CO₂M
- X: C₃-C₁₁-Alkylen und
- M: Wasserstoff, ein Alkali- oder Ammonium-Ion oder das Äquivalent eines Erdalkali-Ions bedeuten.

Beispiele für derartige bevorzugte Verbindungen, die in den erfindungsgemäßen Granulaten eingesetzt werden, sind ε-Phthalimidoperoxihexansäure (PAP), ε-[Dodecylsuccinimido] peroxihexansäure, γ-Phthalimidoperoxibuttersäure und ε-Trimellitimidoperoxihexansäure, deren Salze oder ihre Gemische.

Die Imidoperoxicarbonsäuren können z.B. gemäß EP-349940 hergestellt werden, beispielsweise durch Umsetzung eines Anhydrids der Formel 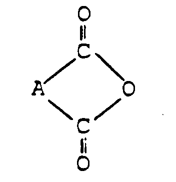 mit Aminosäuren der Formel 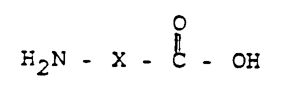 und Oxidation der dabei erhaltenen Imidocarbonsäure mit Wasserstoffperoxid in Gegenwart einer starken Säure. Gemäß einer Variante dieses Verfahrens kann man das Anhydrid auch mit einem Lactam in Gegenwart von Wasser unter Druck umsetzen.

Die Konzentration dieser Persäuren im Granulat beträgt mindestens 60, vorzugsweise 65 - 90 %.

Die zur Granulierung eingesetzten Imidoperoxicarbonsäuren sind normalerweise bei Raumtemperatur Feststoffe mit einem Schmelzpunkt von über 60°C. Sie können in Pulverform, in trockenem oder feuchtem Zustand zur Granulierung eingesetzt werden.

### Das Granulierhilfsmittel

Die Granulierhilfsmittel haben die Aufgabe, durch Agglomerisation mit der Peroxicarbonsäure einen mechanisch stabilen Granulatkern und damit das Grundgerüst des eigentlichen Granulates zu bilden.

Die erfindungsgemäß zu verwendenden Granulierhilfsmittel lassen sich in zwei Gruppen einteilen: a) anorganische Sulfate und/oder Phosphate und b) organische Verbindungen mit oberflächenaktiven Eigenschaften (Tenside). Voraussetzung ist, daß diese Substanzen nicht durch die Persäure oxidiert werden können.

Als anorganische Sulfate/Phosphate eignen sich für die Granulate Sulfate/Phosphate von Alkali- oder Erdalkalimetallen, die gut wasserlöslich sind und nach dem Auflösen neutral oder sauer reagieren. Vorzugsweise verwendet werden Natriumsulfat, Natriumhydrogensulfat, Kaliumsulfat, Kaliumhydrogensulfat, Natriumdihydrogenphosphat oder Magnesiumsulfat. Des weiteren können Mischungen der Salze eingesetzt werden.

Als oberflächenaktive Substanzen werden bevorzugt wasserlösliche anionische Sulfate oder Sulfonate oder zwitterionische Tenside eingesetzt. Beispiele für derartige Verbindungen sind Alkali- oder Erdalkalimetallsalze von Alkylsulfaten oder -sulfonaten mit einer Alkylgruppe von 9 bis 22 Kohlenstoffatomen, welche aus natürlichen oder synthetisch hergestellten Fettalkoholen erhalten werden, oder aus Kohlenwasserstoffen wie z.B. Paraffin. Weitere brauchbare Tenside, die Verwendung finden können, sind Salze von Alkylbenzolsulfonaten, bei denen die Alkylgruppe 9 bis 22 Kohlenstoffe enthält und verzweigt oder unverzweigt sein kann. Alle genannten Verbindungen können ggf. ethoxilierte Gruppen im Molekül tragen. Bevorzugte Verbindungen sind sek. Alkansulfonate, (Hostapur®SAS), Alkylsulfate und Alkylbenzolsufonate.

Die Substanzen können in fester oder pastöser Form oder als Lösung für die Granulierung eingesetzt werden. Bevorzugtes Lösemittel ist in diesem Falle Wasser.

Mischungen der Granulierhilfsmittel der Gruppe a) können in jedem Verhältnis mit denen der Gruppe b) für die Granulierung eingesetzt werden.

Der Anteil des Granulierhilfsmittels im fertigen Granulat beträgt 5 bis 39, vorzugsweise 15 bis 35 Gew.-%.

### Die filmbildende Hüllsubstanz

Als filmbildende Hüllsubstanz werden Polymere der (Meth)Acrylsäure oder Copolymere dieser Säuren mit anderen ungesättigten organischen Carbonsäuren verwendet. Diese Verbindungen können auch in teilneutralisierter Form eingesetzt werden. Von Bedeutung ist jedoch, daß der pH-Wert der Verbindungen zwischen 2,5 und 7 liegt bzw. eingestellt wird. Mögliche Polymere sind beispielsweise Polyacrylsäure, Polymethacrylsäure sowie Copolymere aus Acrylsäure oder Methacrylsäure mit Maleinsäure, Fumarsäure oder Itaconsäure. Die Verbindungen haben ein durchschnittliches Molekulargewicht von 800 - 2.000.000, vorzugsweise 2.000 - 500.000.

Die polymeren Filmbildner werden bevorzugt in wäßriger Lösung auf den Granulatkern aufgebracht. Ihre Konzentration in der Lösung beträgt 5 - 50 %, bevorzugt 10 - 30 %.

Der Anteil der filmbildenden Substanz am Granulat beträgt 1 bis 15, vorzugsweise 3 - 12 %.

### Zusätzliche Komponenten

In manchen Fällen kann es erwünscht sein, daß das erfindungsgemäße Granulat bestimmte zusätzliche Komponenten enthält. Beispiele dafür sind chelatbildende Systeme, Farbstoffe und Mittel zur Regulierung des pH-Wertes.

Es ist bekannt, daß Metallionen fähig sind, organische oder anorganische Perverbindungen katalytisch zu zersetzen. Zur Überwindung dieses Problems kann dem Granulat bis zu 3 % eines Chelatbildners zugesetzt werden. Bevorzugte Verbindungen sind anorganische oder organische Phosphate oder Phosphonate oder Aminomethylencarbonsäuren. Beispiele dafür sind Ethylendiamintetramethylenphosphon- oder -carbonsäuren oder Diethylentriaminpentamethylenphosphonsäure oder deren Salze.

Mittel zur Einstellung des pH-Wertes werden zur Veränderung oder Aufrechterhaltung des pH-Wertes innerhalb des Granulates eingesetzt. Beispiele dafür sind Zitronensäure, Fettsäuren oder Bernsteinsäure oder Salze wie Silikate, Phosphate oder Natriumbisulfat.

### Die Herstellung

Die Imidoperoxicarbonsäure und die Granulierhilfsmittel des Typs a) und/oder b) werden in einem ersten Schritt so vermischt, daß durch Agglomeration geeignete Granulate entstehen. Dies kann in einem Kneter oder Mischer erfolgen. Die Verwendung eines Kneters ist überall dort angebracht, wo durch Zugabe eines pastösen Granulierhilfsmittels eine intensive mechanische Durchmischung notwendig ist. Wird das Vermischen in einem Kneter, z.B. einem Brabender-Kneter, vorgenommen, so hat es sich als günstig erwiesen, das erhaltene Material noch zusätzlich in einem Granulator, z.B. einem Eirich-Granulator, zu verdichten. Werden als Granulierhilfsmittel anorganische, hydratisierbare Salze verwendet, so ist es vorteilhaft, die Imidoperoxicarbonsäure mit einem Wassergehalt von 50 bis 5, vorzugsweise 35 - 20 %, einzusetzen. In diesem Fall kann das Vermischen z.B. in einem Pflugschar-Mischer der Fa. Lödige vorgenommen werden. Die so erhaltenen Granulate bedürfen nach ihrer Trocknung keiner weiteren Kompaktierung. Üblicherweise werden Granulate mit einer Körngröße von 0,5 bis 2 mm angestrebt. Dies kann durch Sieben des Granulats erreicht werden. Der Anteil an Gutkorn liegt im allgemeinen bei 80 %. Die darüber- oder darunterliegenden Anteile können in den Granulierprozess zurückgeführt werden.

Auf die so hergestellte Imidoperoxicarbonsäuregranulate wird in einem Zweiten Schritt die wäßrige Lösung der filmbildenden Hüllsubstanz aufgesprüht. Um einen möglichst vollständigen Überzug zu erhalten, müssen die Granulate während des Aufsprühens bewegt werden. Eine besonders bevorzugte Form ist deshalb das Aufsprühen in einem Wirbelbett. Dabei kann gleichzeitig durch Erwärmen der Wirbelluft das überzogene Granulat getrocknet werden. Das Aufsprühen erfolgt so, daß eine weitere Agglomeration verhindert wird. Korngröße und Korngrößenverteilung werden deshalb durch den Umhüllungsprozeß nur unwesentlich beeinflußt. Zusätzlich können in der wäßrigen Polymerlösung noch Chelatbildner, Farbstoffe und Mittel zur Regulierung des pH-Wertes gelöst sein. Die umhüllten Granulate müssen je nach Ansprühprozess noch getrocknet werden.

Die erfindungsgemäßen Granulate sind weiße, freifließende Granulate mit einem Schüttgewicht zwischen 500 und 1200 kg/m³, vorzugsweise zwischen 550 und 1100 kg/m³.

Eine Nachbehandlung, z.B. Verpressung zu Tabletten oder größeren Agglomeraten ist möglich und für besondere Einsatzzwecke zweckmäßig.

### Verwendung

Die erfindungsgemäßen Granulate lassen sich generell überall dort verwenden, wo die Imidoperoxicarbonsäuren als Oxidations-, Bleich- und Desinfektionsmittel Verwendung finden. Insbesondere können diese Granulate in pulverförmigen Wasch-, Reinigungs- und Desinfektionsmitteln eingesetzt werden. Ein weiteres bevorzugtes Anwendungsgebiet findet sich im Hygienesektor, so beispielsweise als Zusatz zu Desinfektionsmitteln oder Reinigungsmitteln für harte Oberflächen, Sanitärreinigern, Zahnpflegemitteln, oder Fleckensalzen. Durch die Granulierung wird die Lösegeschwindigkeit der Peroxicarbonsäure nicht oder nur unwesentlich beeinflußt. Bei 20°C stehen innerhalb von 5 min mehr als 70 % des verfügbaren Aktivsauerstoffes zum Bleichen, Oxidieren oder Desinfizieren zur Verfügung. Daher wird eine effektive Wirkung der Persäure bereits bei Raumtemperatur erzielt.

Die Granulate können für diesen Zweck mit anderen festen Wirksubstanzen, die im entsprechenden Anwendungsgebiet benötigt werden, konfektioniert werden. Insbesondere sei darauf hingewiesen, daß auch Kombinationen mit anderen Bleichmitteln wie Persalzen, Persalz/Aktivatorsystemen oder anderen Peroxicarbonsäuren in manchen Fällen bevorzugt werden.

Als zusätzliche Komponenten für den Einsatz in Wasch- und Reinigungsmitteln sind anionische, nichtionische oder kationische Tenside, Buildersysteme auf Zeolith-, Schichtsilikat- oder Phosphatbasis, Co-Builder, optische Aufheller und Parfümstoffe zu nennen.

### Beispiel 1

In einem 0,3 l Brabender-Kneter werden 140 g ε-Phthalimidoperoxihexansäure mit 47 g sek. Natriumalkansulfonat (Hostapur® SAS 60) 5 Minuten mit 100 Umdrehungen pro Minute geknetet. Das gesamte Material aus drei Kneteransätzen wird dann in einem 12 Liter Eirich-Mischgranulator mit 900 Umdrehungen pro Minute 2 Minuten granuliert und anschließend im Vakuumtrockenschrank bei 40°C bis zur Gewichtskonstanz getrocknet. Nach Sieben werden 85 % Gutkorn zwischen 0,5 und 2,00 mm erhalten. 337 g Gutkorn werden in einer Wirbelschichtanlage vorgelegt und durch einen 40°C warmen Luftstrom von ca. 50 m³/h aufgewirbelt. Gleichzeitig wird durch eine am Boden befindliche Düse eine wäßrige 25,3 %ige Polyacrylsäurelösung (M_{w} ~ 15000 nach GPC-Analyse), in der zusätzlich 0,24 % Diethylentriaminpentamethylenphosphonsäure (Dequest® 2066 Monsanto) gelöst sind, aufgesprüht. Innerhalb von 36 Minuten werden 176 g Polyacrylsäurelösung auf das bewegte Granulat aufgesprüht. Nach Trocknen im Vakuumtrockenschrank bei 40°C resultieren 351 g umhülltes Granulat mit folgender Zusammensetzung: 69 % ε-Phthalimidoperoxihexansäure (entsprechend einem Aktivsauerstoffgehalt des Granulates von 3,98 %), 18 % sekundäres Natriumalkansulfonat (bestimmt durch Zweiphasentitration nach Epton), 11,8 % Polyacrylsäure. Das Schüttgewicht beträgt 604 g/l.

### Waschversuche

Für die Waschversuche wurden ε-Phthalimidoperoxihexansäure (PAP) als Pulver (Gehalt: 96 %) und das erfindungsgemäße Granulat nach dem vorstehenden Beispiel 1 sowie ein Granulat auf Basis von Laurinsäure eingesetzt.
- Granulat 1: nach Beispiel 1: 69 % PAP, 18 % Alkansulfonat-Na, 11,8 % Polyacrylsäure
- Granulat 2:: nicht erfindungsgemäßes PAP-Granulat auf Basis von Laurinsäure

Die Waschversuche wurden im Launder-O-Meter unter Verwendung der Testanschmutzungen Tee auf Baumwolle (WFK) und Rotwein auf Baumwolle (EMPA, St. Gallen, CH) durchgeführt. Die Wasserhärte betrug 15°dH. Als Waschmittel wurden 1,5 g/l phosphatfreies Standard-Waschmittel (WFK) eingesetzt. Die Menge an Bleichmittel wurde so gewählt, daß theoretisch jeweils 25 mg Aktivsauerstoff pro Liter Waschflotte zur Verfügung standen. Die Waschtemperatur betrug 20°C, die Waschzeit 30 min.

Die Bleichleistung wurde als Remissionszunahme an den verschiedenen Testgeweben bestimmt. Die Auswertung erfolgte in gewohnter Weise.

| Bleichmittel | Remission [%] | |
|---|---|---|
| | Tee | Rotwein |
| PAP-Pulver | 65,6 | 55,6 |
| Granulat 1 | 64,9 | 54,9 |
| Granulat 2 | 60,4 | 51,8 |

Die Waschergebnisse zeigen, daß durch die erfindungsgemäße Granulierung das Aktivsauerstoff-Freisetzungsvermögen der Peroxicarbonsäure bei niederer Temperatur nur wenig beeinflußt wird. Das nicht erfindungsgemäße Granulat 2 hingegen führt wegen verminderter Kaltwasserlöslichkeit zu deutlich schlechteren Bleichergebnissen.

### Lagerversuche

### Bestimmung der Lagerstabilität

Je 100 mg des Granulats nach Beispiel 1 wurden mit 900 mg phosphatfreiem Standard-Waschmittel vermischt und in offenen Glasfläschchen bei 20°C/60 % Luftfeuchte, 38°C/30 % Luftfeuchte und 38°C/80 % Luftfeuchte gelagert. Nach jeweils einer Woche wird der Aktivsauerstoffgehalt einer gesamten Probe bestimmt und das Ergebnis in Bezug zum Ausgangswert gesetzt.

### Lagerstabilität

Erhaltungsgrad des Aktivsauerstoffs in Prozent des ursprünglichen Gehalts.

| Bedingung | Lagerzeit/Wochen | | | |
|---|---|---|---|---|
| | 1 | 2 | 3 | 6 |
| 20°C/60 % LF | 100 | 100 | 100 | 100 |
| 38°C/30 % LF | 100 | 100 | 95 | 97 |
| 38°C/80 % LF | 97 | 87 | 55 | 20 |

## Patentansprüche (Patentansprüche für folgende(n) Vertragsstaat(en): BE, DE, FR, GB, IT, NL)

1. Stabile Peroxicarbonsäuregranulate, bestehend im wesentlichen aus einer Imidoperoxicarbonsäure oder deren Salze der Formel. 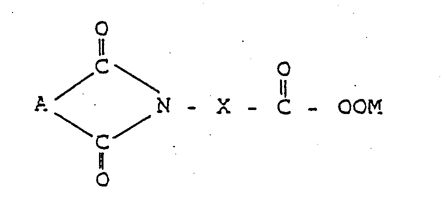 worin
A eine Gruppe der Formeln 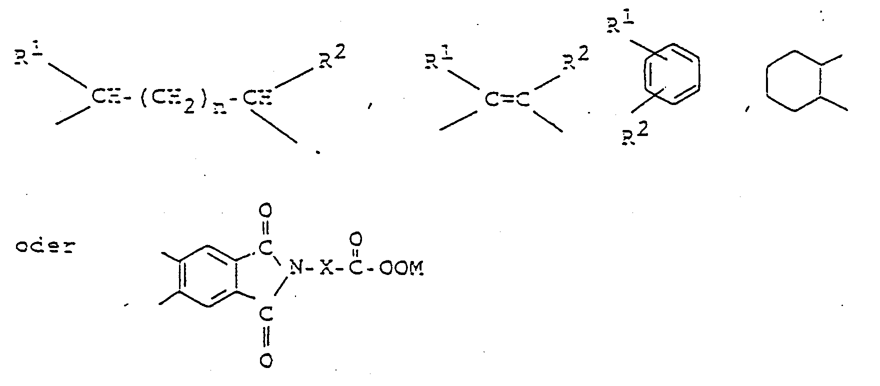
n die Zahlen 0, 1 oder 2,
R¹ Wasserstoff, Chlor, Brom, C₁-C₂₀-Alkyl, Alkenyl mit bis zu 20 C-Atomen, Aryl oder Alkylaryl,
R² Wasserstoff, Chlor, Brom oder eine Gruppe der Formel -SO₃M, -CO₂M oder -OSO₃M,
M Wasserstoff, ein Alkali- oder Ammonium-Ion oder das Äquivalent eines Erdalkali-Ions, und
X C₁-C₁₉-Alkylen oder Arylen bedeuten,
einem anorganischen Sulfat- und/oder Phosphatsalz und/oder einem nicht oxidierbaren Tensid als Granulierhilfsmittel und einem Homo- oder Copolymer aus Einheiten von Acrylsäure, Methacrylsäure und/oder deren Salzen und/oder einem Copolymer aus Einheiten von Acrylsäure, Methacrylsäure und/oder deren Salzen und anderen, hiermit polymerisierbaren Carbonsäuren als filmbildende Hüllsubstanz, wobei der Gehalt an Imidoperoxicarbonsäure in dem Granulat mindestens 60 Gew.% beträgt.

2. Stabile Peroxicarbonsäuregranulate nach Anspruch 1, dadurch gekennzeichnet, daß als Peroxicarbonsäure eine Verbindung der Formel 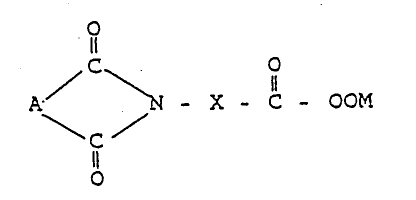 oder deren Salze eingesetzt wird, wobei
A eine Gruppe der Formel
-CH₂-(CH₂)ₙ-CH₂-, -CH₂-CHR¹- oder 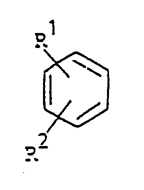
n die Zahlen 0 oder 1,
R¹ Wasserstoff, C₁-C₂₀-Alkyl oder C₁-C₂₀-Alkenyl,
R² Wasserstoff oder CO₂M
X C₃-C₁₁-Alkylen und
M Wasserstoff, ein Alkali- oder Ammonium-Ion oder das Äquivalent eines Erdalkali-Ions bedeuten.

3. Stabile Peroxicarbonsäuregranulate nach Anspruch 1, dadurch gekennzeichnet, daß als Granulierhilfsmittel Natriumsulfat und/oder Alkylbenzolsulfonat, Alkansulfonat oder Alkylsulfat verwendet werden.

4. Stabile Peroxicarbonsäuregranulate nach Anspruch 1, dadurch gekennzeichnet, daß als polymere Hüllsubstanz Polyacrylsäure oder Copolymere aus Acrylsäure oder Methacrylsäure mit Maleinsäure, Fumarsäure oder Itakonsäure eingesetzt werden.

5. Stabile Peroxicarbonsäuregranulate nach Anspruch 1, dadurch gekennzeichnet, daß der Anteil des Granulierhilfsmittels im fertigen Granulat 5 bis 39 % beträgt.

6. Stabile Peroxicarbonsäuregranulate nach Anspruch 1, dadurch gekennzeichnet, daß der Anteil an polymerer Hüllsubstanz 1 bis 15 % beträgt.

7. Stabile Peroxicarbonsäuregranulate nach Anspruch 1, dadurch gekennzeichnet, daß sie zusätzlich eine nicht polymere chelatkomplexbildende Substanz zur Komplexierung von Schwermetallen enthalten.

8. Stabile Peroxicarbonsäuregranulate nach Anspruch 1, dadurch gekennzeichnet, daß R¹ Phenyl oder C₁-C₄-Alkylphenyl bedeutet.

9. Stabile Peroxicarbonsäuregranulate nach Anspruch 1, dadurch gekennzeichnet, daß X C₃-C₁₁-Alkylen oder Phenylen bedeutet.

10. Stabile Peroxicarbonsäuregranulate nach Anspruch 1, dadurch gekennzeichnet, daß der Anteil des Granulierhilfsmittels im fertigen Granulat 15 bis 35 % beträgt.

11. Stabile Peroxicarbonsäuregranulate nach Anspruch 1, dadurch gekennzeichnet, daß der Anteil an polymerer Hüllsub stanz 3 bis 12 % beträgt.

12. Verwendung von Granulaten gemäß Anspruch 1 als Bleich-, Oxidations- und Desinfektionsmittel.

13. Verwendung von Granulaten gemäß Anspruch 1 in Wasch-, Reinigungs- und Desinfektionsmitteln.

## Patentansprüche (Patentansprüche für folgende(n) Vertragsstaat(en): ES)

1. Wasch-, Reinigungs- und/oder Desinfektionsmittel, enthaltend stabile Peroxicarbonsäuregranulate, bestehend im wesentlichen aus einer Imidoperoxicarbonsäure oder deren Salze der Formel. 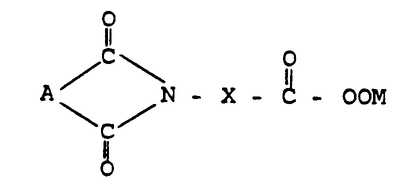 worin
A eine Gruppe der Formeln 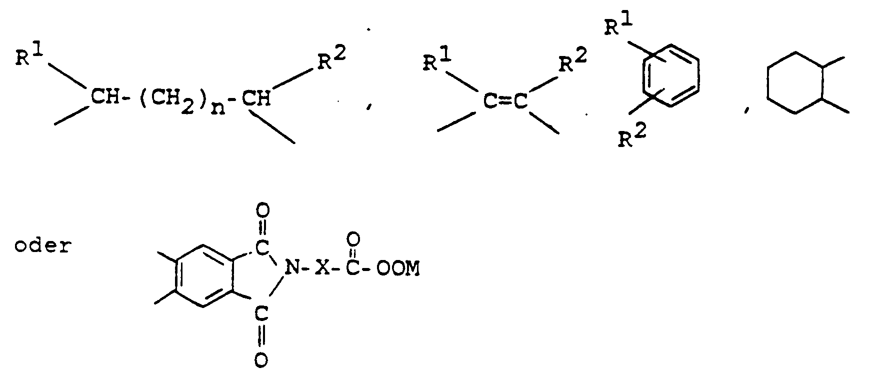
n die Zahlen 0, 1 oder 2,
R¹ Wasserstoff, Chlor, Brom, C₁-C₂₀-Alkyl, Alkenyl mit bis zu 20 C-Atomen, Aryl, oder Alkylaryl,
R² Wasserstoff, Chlor, Brom oder eine Gruppe der Formel -SO₃M, -CO₂M oder -OSO₃M,
M Wasserstoff, ein Alkali- oder Ammonium-Ion oder das Äquivalent eines Erdalkali-Ions, und
X C₁-C₁₉-Alkylen, bevorzugt C₃-C₁₁-Alkylen oder Arylen
einem anorganischen Sulfat- und/oder Phosphatsalz und/oder einem nicht oxidierbaren Tensid als Granulierhilfsmittel und einem Homo- oder Copolymer aus Einheiten von Acrylsäure, Methacrylsäure und/oder deren Salzen und/oder einem Copolymer aus Einheiten von Acrylsäure, Methacrylsäure und/oder deren Salzen und anderen, hiermit polymerisierbaren Carbonsäuren als filmbildende Hüllsubstanz wobei der Gehalt an Imidoperoxicarbonsäure in dem Granulat mindestens 60 Gew.% beträgt.

## Claims (Claims for the following Contracting State(s): BE, DE, FR, GB, IT, NL)

1. A stable peroxycarboxylic acid granule, composed essentially of an imidoperoxycarboxylic acid or its salts of the formula 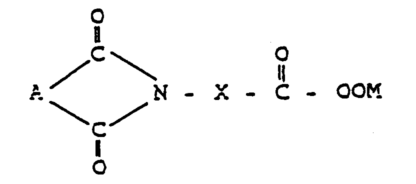 in which
A is a group of the formula 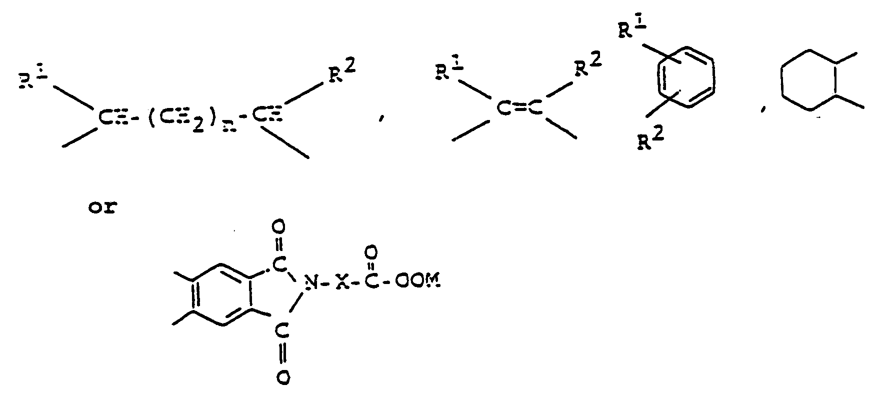
n is the number 0, 1 or 2,
R¹ is hydrogen, chlorine, bromine, C₁-C₂₀-alkyl, alkenyl having up to 20 carbon atoms, aryl or alkylaryl,
R² is hydrogen, chlorine, bromine or a group of the formula -SO₃M, -CO₂M or -OSO₃M,
M is hydrogen, an alkali metal or ammonium ion or the equivalent of an alkaline earth metal ion, and
X is C₁-C₁₉-alkylene or arylene,
an inorganic sulfate and/or phosphate salt and/or a non-oxidizable surfactant as a granulating auxiliary and a homo- or copolymer of units of acrylic acid, methacrylic acid and/or their salts and/or a copolymer of units of acrylic acid, methacrylic acid and/or their salts and other carboxylic acids polymerizable therewith as a film-forming coating substance, the content of imidoperoxycarboxylic acid in the granule being at least 60% by weight.

2. A stable peroxycarboxylic acid granule as claimed in claim 1, wherein, as a peroxycarboxylic acid, a compound of the formula 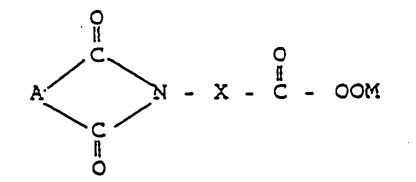 or its salts is employed, in which
A is a group of the formula
-CH₂-(CH₂)ₙ-CH₂-, -CH₂-CHR¹- or 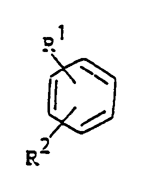
n is the number 0 or 1,
R¹ is hydrogen, C₁-C₂₀-alkyl or C₁-C₂₀-alkenyl,
R² is hydrogen or CO₂M,
X is C₃-C₁₁-alkylene and
M is hydrogen, an alkali metal or ammonium ion or the equivalent of an alkaline earth metal ion.

3. A stable peroxycarboxylic acid granule as claimed in claim 1, wherein sodium sulfate and/or alkylbenzene-sulfonate, alkanesulfonate or alkylsulfate are used as the granulating auxiliary.

4. A stable peroxycarboxylic acid granule as claimed in claim 1, wherein polyacrylic acid or copolymers of acrylic acid or methacrylic acid with maleic acid, fumaric acid or itaconic acid are employed as the polymeric coating substance.

5. A stable peroxycarboxylic acid granule as claimed in claim 1, wherein the amount of the granulating auxiliary in the finished granule is 5 to 39%.

6. A stable peroxycarboxylic acid granule as claimed in claim 1, wherein the amount of polymeric coating substance is 1 to 15%.

7. A stable peroxycarboxylic acid granule as claimed in claim 1, which additionally contains a nonpolymeric chelate complex-forming substance to complex heavy metals.

8. A stable peroxycarboxylic acid granule as claimed in claim 1, wherein R¹ is phenyl or C₁-C₄-alkylphenyl.

9. A stable peroxycarboxylic acid granule as claimed in claim 1, wherein X is C₃-C₁₁-alkyllene or phenylene.

10. A stable peroxycarboxylic acid granule as claimed in claim 1, wherein the amount of the granulating auxiliary in the finished granule is 15 to 35%.

11. A stable peroxycarboxylic acid granule as claimed in claim 1, wherein the amount of polymeric coating substance is 3 to 12%.

12. The use of granules as claimed in claim 1 as bleaching agents, oxidizing agents and disinfectants.

13. The use of granules as claimed in claim 1 in detergents, cleaning agents or disinfectants.

## Claims (Claims for the following Contracting State(s): ES)

1. A detergent, cleaning agent and/or disinfectant containing a stable peroxycarboxylic acid granule, composed essentially of an imidoperoxycarboxylic acid or its salts of the formula 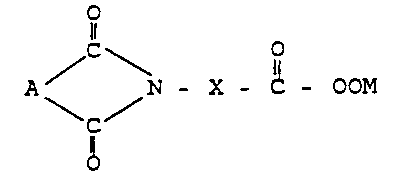 in which
A is a group of the formula 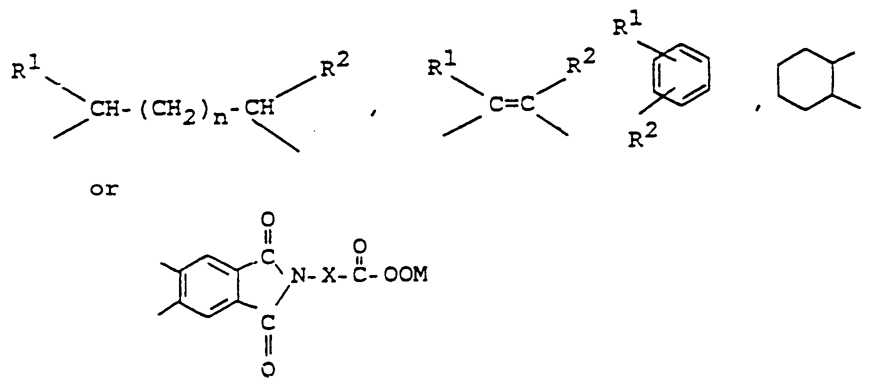
n is the number 0, 1 or 2,
R¹ is hydrogen, chlorine, bromine, C₁-C₂₀-alkyl, alkenyl having up to 20 carbon atoms, aryl or alkylaryl,
R² is hydrogen, chlorine, bromine or a group of the formula -SO₃M, -CO₂M or -OSO₃M,
M is hydrogen, an alkali metal or ammonium ion or the equivalent of an alkaline earth metal ion, and
X is C₁-C₁₉-alkylene, or arylene,
an inorganic sulfate and/or phosphate salt and/or a non-oxidizable surfactant as a granulating auxiliary and a homo- or copolymer of units of acrylic acid, methacrylic acid and/or their salts and/or a copolymer of units of acrylic acid, methacrylic acid and/or their salts and other carboxylic acids polymerizable therewith as a film-forming coating substance, the content of imidoperoxycarboxylic acid in the granule being at least 60% by weight.

## Revendications (Revendications pour l'(les) Etat(s) contractant(s) suivant(s): BE, DE, FR, GB, IT, NL)

1. Granulés stables d'acide peroxycarboxylique constitués essentiellement d'un acide imidoperoxycarboxylique ou de ses sels de formule 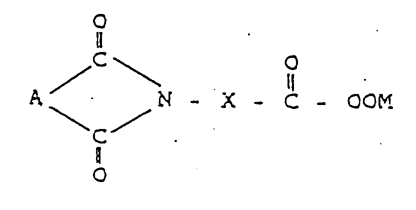 où
A est un groupe de formules 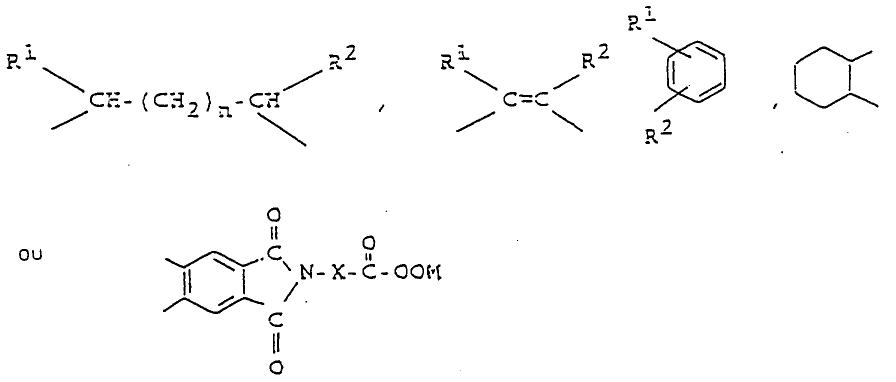
n vaut 0, 1 ou 2,
R¹ représente l'hydrogène, le chlore, le brome, alkyle en C₁-C₂₀, alcényle avec jusqu'à 20 atomes de carbone, aryle ou alkylaryle,
R² représente l'hydrogène, le chlore, le brome ou un groupe de formule -SO₃M, -CO₂M ou -OSO₃M,
M représente l'hydrogène, un ion alcali ou ammonium ou l'équivalent d'un ion alcalino-terreux et
X représente alkylène en C₁-C₁₉ ou arylène,
avec un sel sulfate et/ou phosphate inorganique et/ou un agent de surface non oxydable en tant qu'adjuvant de granulation et un homo- ou copolymère de motifs d'acide acrylique, d'acide (méth)acrylique et/ou de leurs sels, et/ou d'un copolymère de motifs d'acide acrylique, d'acide (méth)acrylique et/ou de leurs sels et d'autres acides carboxyliques organiques polymérisables de cette façon comme substances d'enrobage filmogène, la teneur en acide imidoperoxycarboxylique dans le granulé est d'au moins 60 % en poids.

2. Granulés d'acide peroxycarboxylique stables selon la revendication 1, caractérisés en ce que l'on utilise comme acide peroxycarboxylique un composé de formule 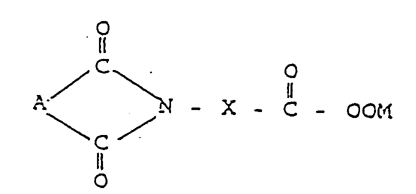 ou ses sels,
A représente un groupe de formule
-CH₂-(CH₂)ₙ-CH₂- ou -CH₂-CHR¹-, 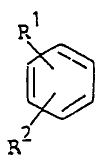
n vaut 0 ou 1,
R¹ représente l'hydrogène, alkyle en C₁-C₂₀ ou alcényle en C₁-C₂₀,
R² représente l'hydrogène ou CO₂M,
X représente alkylène en C₃-C₁₁ et
M représente l'hydrogène, un ion alcali ou ammonium ou l'équivalent d'un ion alcalino-terreux.

3. Granulés stables d'acide peroxycarboxylique selon la revendication 1, caractérisés en ce que l'on utilise comme adjuvant de granulation du sulfate de sodium et/ou de l'alkylbenzènesulfonate, de l'alcanesulfonate ou de l'alkylsulfate.

4. Granulés stables d'acide peroxycarboxylique selon la revendication 1, caractérisés en ce que l'on utilise comme substance d'enrobage polymère l'acide polyacrylique ou des copolymères de l'acide acrylique ou de l'acide méthacrylique avec l'acide maléique, l'acide fumarique ou l'acide itaconique.

5. Granulés stables d'acide peroxycarboxylique selon la revendication 1, caractérisés en ce que le taux en adjuvant de granulation dans le granulat fini est de 5 ) 39 %.

6. Granulés stables d'acide peroxycarboxylique selon la revendication 1, caractérisés en ce que le taux en substance d'enrobage polymère est de 1 à 15 %.

7. Granulés stables d'acide peroxycarboxylique selon la revendication 1, caractérisés en ce qu'ils contiennent de plus une substance chélatante complexante non polymère pour complexer des métaux lourds.

8. Granulés stables d'acide peroxycarboxylique selon la revendication 1, caractérisés en ce que R¹ représente phényle ou (alkyle en C₁-C₄)phényle.

9. Granulés stables d'acide peroxycarboxylique selon la revendication 1, caractérisés en ce que X représente alkylène en C₃-C₁₁ ou phénylène.

10. Granulés stables d'acide peroxycarboxylique selon la revendication 1, caractérisés en ce que le taux en adjuvant de granulation dans le granulat fini est de 15 à 35 %.

11. Granulés stables d'acide peroxycarboxylique selon la revendication 1, caractérisés en ce que le taux en substance d'enrobage polymère est de 3 à 12 %.

12. Utilisation de granulés selon la revendication 1 comme agent de blanchiment, d'oxydation et de désinfection.

13. Utilisation des granulés selon la revendication 1 dans les produits de lavage, de nettoyage et de désinfection.

## Revendications (Revendications pour l'(les) Etat(s) contractant(s) suivant(s): ES)

1. Produits de lavage, de nettoyage et/ou de désinfection contenant des granulés stables d'acide peroxycarboxylique, constitués essentiellement d'un acide imidoperoxycarboxylique ou de ses sels de formule 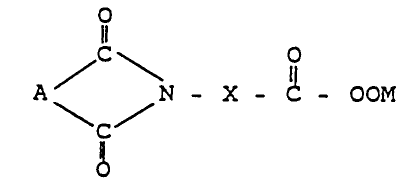 où
A est un groupe de formules 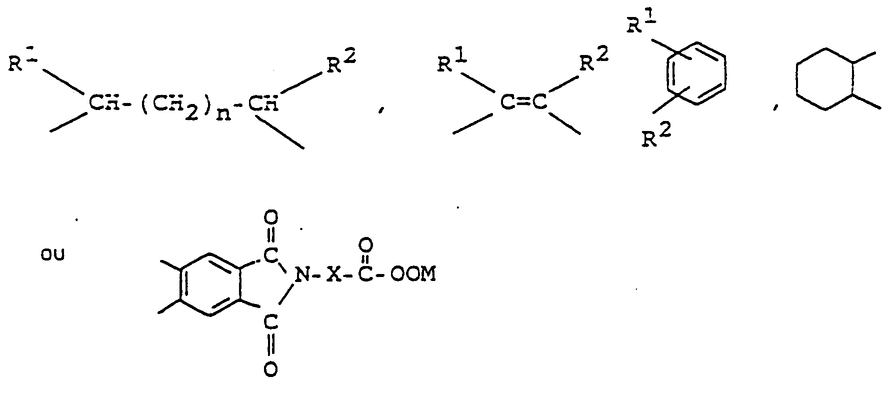
n vaut 0, 1 ou 2,
R¹ représente l'hydrogène, le chlore, le brome, alkyle en C₁-C₂₀, alcényle avec jusqu'à 20 atomes de carbone, aryle ou alkylaryle,
R² représente l'hydrogène, le chlore, le brome ou un groupe de formule -SO₃M, -CO₂M ou -OSO₃M,
M représente l'hydrogène, un ion alcali ou ammonium ou l'équivalent d'un ion alcalino-terreux et
X représente alkylène en C₁-C₁₉, ou arylène,
avec un sel sulfate et/ou phosphate inorganique et/ou un agent de surface non oxydable en tant qu'adjuvant de granulation et un homo- ou copolymère de motifs l'acide acrylique, l'acide (méth)acrylique et/ou de leurs sels, et/ou d'un copolymère de motifs l'acide acrylique, l'acide (méth)acrylique ou de ses sels et d'autres acides carboxyliques organiques polymérisables de cette façon comme substances d'enrobage filmogène, la teneur en acide imidoperoxycarboxylique dans le granulé étant d'au moins 60 % en poids.
